# EUROPEAN PATENT SPECIFICATION

(11) **EP 1 712 560 B1**
(45) Date of publication and mention of the grant of the patent: **14.09.2011**
(21) Application number: 06007819.3
(22) Date of filing: 13.04.2006
(51) Int. Cl.: C07K 14/415, A61K 38/00

(54) **Hypoallergenic proteins derived from the major allergen of parietaria judaica**
Hypoallergene Proteine abgeleitet vom Hauptallergen von Parietaria judaica
Protéines hypoallergéniques dérivés de l'allergène principal de Parietaria judaica

(30) Priority: 15.04.2005 IT MI20050669
(43) Date of publication of application: 18.10.2006
(73) Proprietor: Lofarma S.p.A., 20143 Milano (IT)
(72) Inventor: Mistrello, Giovanni, 20143 Milano (IT); Zanotta, Stefania, 20143 Milano (IT); Roncarolo, Daniela, 20143 Milano (IT); Falagiani, Paolo, 20143 Milano (IT); Viotti, Angelo, 00100 Roma (IT)
(74) Representative: Banfi, Paolo

(56) References cited:
- WO-A-02/22674
- DATABASE Geneseq [Online] 24 February 2005 (2005-02-24), "Parietaria judaica major allergen Par j 1 hypoallergenic variant." XP002391042 retrieved from EBI accession no. GSN:ADV25389 Database accession no. ADV25389 -& WO 2004/104047 A (COSTA, MARIA, ASSUNTA; GERACI, DOMENICO; COLOMBO, PAOLO; PASSANTINO, R) 2 December 2004 (2004-12-02)
- COLOMBO P ET AL: "Identification of an immunodominant IgE epitope of the Parietaria judaica major allergen" JOURNAL OF IMMUNOLOGY, THE WILLIAMS AND WILKINS CO. BALTIMORE, US, vol. 160, no. 6, 15 March 1998 (1998-03-15), pages 2780-2785, XP002085252 ISSN: 0022-1767
- BONURA A ET AL: "HYPOALLERGENIC VARIANTS OF THE PARIETARIA JUDAICA MAJOR ALLERGEN PAR J 1: A MEMBER OF THE NON-SPECIFIC LIPID TRANSFER PROTEIN PLANT FAMILY" INTERNATIONAL ARCHIVES OF ALLERGY AND IMMUNOLOGY, vol. 126, no. 1, September 2001 (2001-09), pages 32-40, XP001037388 ISSN: 1018-2438
- FERREIRA F ET AL: "Modulation of IgE reactivity of allergens by site-directed mutagenesis: potential use of hypoallergenic variants for immunotherapy" FASEB JOURNAL, FED. OF AMERICAN SOC. FOR EXPERIMENTAL BIOLOGY, BETHESDA, MD, US, vol. 12, no. 2, February 1998 (1998-02), pages 231-242, XP002085249 ISSN: 0892-6638

### SEQUENCE LISTING

<110> LOFARMA
<120> hypoallergenic proteins derived from the major allergen of *Parietaria judaica*
<130> 7474Meur
<160> 14
<170> PatentIn version 3.1
<210> 1
   <211> 139
   <212> PRT
   <213> Unknown
<220>
<223> Par j 1 wild type
<400> 1
   Glu Glu Thr Cys Gly Thr Val Val Gly Ala Leu Met Pro Cys Leu Pro
   1 5 10 15
   Phe Val Gln Gly Lys Glu Lys Glu Pro Ser Lys Gly Cys Cys Ser Gly
   20 25 30
   Ala Lys Arg Leu Asp Gly Glu Thr Lys Thr Gly Pro Gln Arg Val His
   35 40 45
   Ala Cys Glu Cys Ile Gln Thr Ala Met Lys Thr Tyr Ser Asp Ile Asp
   50 55 60
   Gly Lys Leu Val Ser Glu Val Pro Lys His Cys Gly Ile Val Asp Ser
   65 70 75 80
   Lys Leu Pro Pro Ile Asp Val Asn Met Asp Cys Lys Thr Leu Gly Val
   85 90 95
   Val Pro Arg Gln Pro Gln Leu Pro Val Ser Leu Arg His Gly Pro Val
   100 105 110
   Thr Gly Pro Ser Asp Pro Ala His Lys Ala Arg Leu Glu Arg Pro Gln
   115 120 125
   Ile Arg Val Pro Pro Pro Ala Pro Glu Lys Ala
   130 135
<210> 2
<211> 139
<212> PRT
<213> Unknown
<220>
<223> Par j 1 mutant
<400> 2
   Glu Glu Thr Cys Gly Thr Val Val Gly Ala Leu Met Pro Cys Leu Pro
   1 5 10 15
   Phe Val Gln Gly Lys Glu Ala Glu Pro Ser Lys Gly Cys Cys Ser Gly
   20 25 30
   Ala Lys Arg Leu Asp Gly Glu Thr Lys Thr Gly Pro Gln Arg Val His
   35 40 45
   Ala Cys Glu Cys Ile Gln Thr Ala Met Lys Thr Tyr Ser Asp Ile Asp
   50 55 60
   Gly Lys Leu Val Ser Glu Val Pro Lys His Cys Gly Ile Val Asp Ser
   65 70 75 80
   Lys Leu Pro Pro Ile Asp Val Asn Met Asp Cys Lys Thr Leu Gly Val
   85 90 95
   Val Pro Arg Gln Pro Gln Leu Pro Val Ser Leu Arg His Gly Pro Val
   100 105 110
   Thr Gly Pro Ser Asp Pro Ala His Lys Ala Arg Leu Glu Arg Pro Gln
   115 120 125
   Ile Arg Val Pro Pro Pro Ala Pro Glu Lys Ala
   130 135
<210> 3
   <211> 139
   <212> PRT
   <213> Unknown
<220>
<223> Par j 1 mutant
<400> 3
   Glu Glu Thr Cys Gly Thr Val Val Gly Ala Leu Met Pro Cys Leu Pro
   1               5                   10                  15
   Phe Val Gln Gly Lys Glu Ala Glu Pro Ser Ala Gly Cys Cys Ser Gly
               20                  25                  30
   Ala Lys Arg Leu Asp Gly Glu Thr Lys Thr Gly Pro Gln Arg Val His
           35                  40                  45
   Ala Cys Glu Cys Ile Gln Thr Ala Met Lys Thr Tyr Ser Asp Ile Asp
       50                  55                  60
   Gly Lys Leu Val Ser Glu Val Pro Lys His Cys Gly Ile Val Asp Ser
   65                  70                  75                  80
   Lys Leu Pro Pro Ile Asp Val Asn Met Asp Cys Lys Thr Leu Gly Val
                   85                  90                  95
   Val Pro Arg Gln Pro Gln Leu Pro Val Ser Leu Arg His Gly Pro Val
               100                 105                 110
   Thr Gly Pro Ser Asp Pro Ala His Lys Ala Arg Leu Glu Arg Pro Gln
           115                 120                 125
   Ile Arg Val Pro Pro Pro Ala Pro Glu Lys Ala
       130                 135
<210> 4
   <211> 139
   <212> PRT
   <213> Unknown
<220>
<223> Par j 1 mutant
<400> 4
   Glu Glu Thr Cys Gly Thr Val Val Gly Ala Leu Met Pro Cys Leu Pro
   1 5 10 15
   Phe Val Gln Gly Lys Glu Ala Glu Pro Ser Ala Gly Cys Cys Ser Gly
   20 25 30
   Ala Lys Arg Leu Asp Gly Glu Thr Lys Thr Gly Pro Gln Arg Val His
   35 40 45
   Ala Cys Glu Cys Ile Gln Thr Ala Met Lys Thr Tyr Ser Asp Ile Asp
   50 55 60
   Gly Lys Leu Val Ser Glu Val Pro Ala His Cys Gly Ile Val Asp Ser
   65 70 75 80
   Lys Leu Pro Pro Ile Asp Val Asn Met Asp Cys Lys Thr Leu Gly Val
   85 90 95
   Val Pro Arg Gln Pro Gln Leu Pro Val Ser Leu Arg His Gly Pro Val
   100 105 110
   Thr Gly Pro Ser Asp Pro Ala His Lys Ala Arg Leu Glu Arg Pro Gln
   115 120 125
   Ile Arg Val Pro Pro Pro Ala Pro Glu Lys Ala
   130 135
<210> 5
   <211> 139
   <212> PRT
   <213> Unknown
<220>
<223> Par j 1 mutant
<400> 5
   Glu Glu Thr Cys Gly Thr Val Val Gly Ala Leu Met Pro Cys Leu Pro
   1 5 10 15
   Phe Val Gln Gly Lys Glu Lys Glu Pro Ser Lys Gly Cys Cys Ser Gly
   20 25 30
   Ala Lys Arg Leu Asp Gly Glu Thr Ala Ala Gly Pro Gln Ala Val His
   35 40 45
   Ala Cys Glu Cys Ile Gln Thr Ala Met Lys Thr Tyr Ser Asp Ile Asp
   50 55 60
   Gly Lys Leu Val Ser Glu Val Pro Lys His Cys Gly Ile Val Asp Ser
   65 70 75 80
   Lys Leu Pro Pro Ile Asp Val Asn Met Asp Cys Lys Thr Leu Gly Val
   85 90 95
   Val Pro Arg Gln Pro Gln Leu Pro Val Ser Leu Arg His Gly Pro Val
   100 105 110
   Thr Gly Pro Ser Asp Pro Ala His Lys Ala Arg Leu Glu Arg Pro Gln
   115 120 125
   Ile Arg Val Pro Pro Pro Ala Pro Glu Lys Ala
   130 135
<210> 6
   <211> 139
   <212> PRT
   <213> Unknown
<220>
<223> Par j 1 mutant
<400> 6
   Glu Glu Thr Cys Gly Thr Val Val Gly Ala Leu Met Pro Cys Leu Pro
   1 5 10 15
   Phe Val Gln Gly Lys Glu Lys Glu Pro Ser Lys Gly Cys Cys Ser Gly
   20 25 30
   Ala Lys Arg Leu Asp Gly Glu Thr Lys Thr Gly Pro Gln Arg Val His
   35 40 45
   Ala Cys Glu Cys Ile Gln Thr Ala Met Lys Thr Tyr Ser Asp Ile Asp
   50 55 60
   Gly Lys Leu Val Ser Glu Val Pro Lys His Cys Gly Ile Val Asp Ser
   65 70 75 80
   Lys Leu Pro Pro Ile Asp Val Asn Met Asp Cys Ala Thr Leu Gly Val
   85 90 95
   Val Pro Arg Gln Pro Gln Leu Pro Val Ser Leu Arg His Gly Pro Val
   100 105 110
   Thr Gly Pro Ser Asp Pro Ala His Ala Ala Arg Leu Glu Arg Pro Gln
   115 120 125
   Ile Arg Val Pro Pro Pro Ala Pro Glu Ala Ala
   130 135
<210> 7
   <211> 139
   <212> PRT
   <213> Unknown
<220>
<223> Par j 1 mutant
<400> 7
   Glu Glu Thr Cys Gly Thr Val Val Gly Ala Leu Met Pro Cys Leu Pro
   1               5                   10                  15
   Phe Val Gln Gly Lys Glu Ala Glu Pro Ser Ala Gly Cys Cys Ser Gly
               20                  25                  30
   Ala Lys Arg Leu Asp Gly Glu Thr Ala Thr Gly Pro Gln Arg Val His
           35                  40                  45
   Ala Cys Glu Cys Ile Gln Thr Ala Met Lys Thr Tyr Ser Asp Ile Asp
       50                  55                  60
   Gly Ala Leu Val Ser Glu Val Pro Lys His Cys Gly Ile Val Asp Ser
   65                  70                  75                  80
   Lys Leu Pro Pro Ile Asp Val Asn Met Asp Cys Lys Thr Leu Gly Val
                   85                  90                  95
   Val Pro Arg Gln Pro Gln Leu Pro Val Ser Leu Arg His Gly Pro Val
               100                 105                 110
   Thr Gly Pro Ser Asp Pro Ala His Lys Ala Arg Leu Glu Arg Pro Gln
           115                 120                 125
   Ile Arg Val Pro Pro Pro Ala Pro Glu Lys Ala
       130                 135
<210> 8
   <211> 420
   <212> DNA
   <213> Unknown
<220>
<223> Par j 1 mutant (coding sequence)
<400> 8
   gaagaaacct gcgggactgt agtgggagcg ctgatgccgt gcctgccgtt cgtgcagggg 60
   aaagaggcag agccgtcaaa ggggtgctgc agcggcgcca aaagattgga cggggagacg 120
   aagacggggc cgcagagggt gcacgcttgt gagtgcatcc agaccgccat gaagacctat 180
   tccgacatcg acgggaaact cgtcagcgag gtccccaagc actgcggcat cgttgacagc 240
   aagctcccgc ccattgacgt caacatggac tgcaagacac ttggagtggt acctcggcaa 300
   ccccaacttc cagtctctct ccgtcatggt cccgtcacgg gcccaagtga ccccgcccac 360
   aaagcacggt tggagagacc ccagattaga gttccgcccc ccgcaccgga aaaagcctaa 420
<210> 9
   <211> 420
   <212> DNA
   <213> Unknown
<220>
<223> Par j 1 mutant (coding sequence)
<400> 9
   gaagaaacct gcgggactgt agtgggagcg ctgatgccgt gcctgccgtt cgtgcagggg 60
   aaagaggcag agccgtcagc ggggtgctgc agcggcgcca aaagattgga cggggagacg 120
   aagacggggc cgcagagggt gcacgcttgt gagtgcatcc agaccgccat gaagacctat 180
   tccgacatcg acgggaaact cgtcagcgag gtccccaagc actgcggcat cgttgacagc 240
   aagctcccgc ccattgacgt caacatggac tgcaagacac ttggagtggt acctcggcaa 300
   ccccaacttc cagtctctct ccgtcatggt cccgtcacgg gcccaagtga ccccgcccac 360
   aaagcacggt tggagagacc ccagattaga gttccgcccc ccgcaccgga aaaagcctaa 420
<210> 10
   <211> 420
   <212> DNA
   <213> Unknown
<220>
<223> Par j 1 mutant (coding sequence)
<400> 10
   gaagaaacct gcgggactgt agtgggagcg ctgatgccgt gcctgccgtt cgtgcagggg    60
   aaagaggcag agccgtcagc ggggtgctgc agcggcgcca aaagattgga cggggagacg   120
   aagacggggc cgcagagggt gcacgcttgt gagtgcatcc agaccgccat gaagacctat   180
   tccgacatcg acgggaaact cgtcagcgag gtccccgcgc actgcggcat cgttgacagc   240
   aagctcccgc ccattgacgt caacatggac tgcaagacac ttggagtggt acctcggcaa   300
   ccccaacttc cagtctctct ccgtcatggt cccgtcacgg gcccaagtga ccccgcccac   360
   aaagcacggt tggagagacc ccagattaga gttccgcccc ccgcaccgga aaaagcctaa   420
<210> 11
   <211> 420
   <212> DNA
   <213> Unknown
<220>
<223> Par j 1 mutant (coding sequence)
<400> 11
   gaagaaacct gcgggactgt agtgggagcg ctgatgccgt gcctgccgtt cgtgcagggg    60
   aaagagaaag agccgtcaaa ggggtgctgc agcggcgcca aaagattgga cggggagacg   120
   gcggcggggc cgcaggcggt gcacgcttgt gagtgcatcc agaccgccat gaagacctat   180
   tccgacatcg acgggaaact cgtcagcgag gtccccaagc actgcggcat cgttgacagc   240
   aagctcccgc ccattgacgt caacatggac tgcaagacac ttggagtggt acctcggcaa   300
   ccccaacttc cagtctctct ccgtcatggt cccgtcacgg gcccaagtga ccccgcccac   360
   aaagcacggt tggagagacc ccagattaga gttccgcccc ccgcaccgga aaaagcctaa   420
<210> 12
   <211> 420
   <212> DNA
   <213> Unknown
<220>
<223> Par j 1 mutant (coding sequence)
<400> 12
   gaagaaacct gcgggactgt agtgggagcg ctgatgccgt gcctgccgtt cgtgcagggg     60
   aaagagaaag agccgtcaaa ggggtgctgc agcggcgcca aaagattgga cggggagacg    120
   aagacggggc cgcagagggt gcacgcttgt gagtgcatcc agaccgccat gaagacctat    180
   tccgacatcg acgggaaact cgtcagcgag gtccccaagc actgcggcat cgttgacagc    240
   aagctcccgc ccattgacgt caacatggac tgcgcgacac ttggagtggt acctcggcaa    300
   ccccaacttc cagtctctct ccgtcatggt cccgtcacgg gcccaagtga ccccgcccac    360
   gcagcacggt tggagagacc ccagattaga gttccgcccc ccgcaccgga agcagcctaa    420
<210> 13
   <211> 420
   <212> DNA
   <213> Unknown
<220>
<223> Par j 1 mutant (coding sequence)
<400> 13
   gaagaaacct gcgggactgt agtgggagcg ctgatgccgt gcctgccgtt cgtgcagggg     60
   aaagaggcag agccgtcagc ggggtgctgc agcggcgcca aaagattgga cggggagacg    120
   gcgacggggc cgcagagggt gcacgcttgt gagtgcatcc agaccgccat gaagacctat    180
   tccgacatcg acggggcact cgtcagcgag gtccccaagc actgcggcat cgttgacagc    240
   aagctcccgc ccattgacgt caacatggac tgcaagacac ttggagtggt acctcggcaa    300
   ccccaacttc cagtctctct ccgtcatggt cccgtcacgg gcccaagtga ccccgcccac    360
   aaagcacggt tggagagacc ccagattaga gttccgcccc ccgcaccgga aaaagcctaa    420
<210> 14
   <211> 420
   <212> DNA
   <213> Unknown
<220>
<223> Par j 1 wild-type (coding sequence)
<400> 14
   gaagaaacct gcgggactgt agtgggagcg ctgatgccgt gcctgccgtt cgtgcagggg     60
   aaagagaaag agccgtcaaa ggggtgctgc agcggcgcca aaagattgga cggggagacg    120
   aagacggggc cgcagagggt gcacgcttgt gagtgcatcc agaccgccat gaagacctat    180
   tccgacatcg acgggaaact cgtcagcgag gtccccaagc actgcggcat cgttgacagc    240
   aagctcccgc ccattgacgt caacatggac tgcaagacac ttggagtggt acctcggcaa    300
   ccccaacttc cagtctctct ccgtcatggt cccgtcacgg gcccaagtga ccccgcccac    360
   aaagcacggt tggagagacc ccagattaga gttccgcccc ccgcaccgga aaaagcctaa    420

## Description

The present invention provides hypoallergenic proteins derived from the major allergen of *Parietaria judaica* Par j 1, pharmaceutical compositions containing them and their use in the prophylaxis and therapy of allergic diseases caused by pollen of plants of the species *Parietaria.*

### BACKGROUND OF THE INVENTION

Allergies are caused by a dysfunction in the immune system, which reacts by producing IgE-class antibodies against proteins contained mainly in pollen, mites, epithelia, and some foods, which proteins are *per se* completely innocuous.

Recent estimations indicate that above 10% of the population in Western countries suffers from this disease, which may induce a worsening of symptoms (e.g. appearance of asthma) and a sensitization to other allergens with the time, thus making more complicated the choice of an appropriate therapy.

Specific immunotherapy (SIT), contrary to pharmacological therapy, is the only kind of etiological treatment of allergic diseases capable of favourably affecting some immunological parameters which are the basis of these diseases.

SIT consists of the administration of increasing doses of standardized extracts (vaccines) obtained from the same substance which causes the disease.

In this way, a sort of immunological tolerance to said substance gradually increases in the patient, which is accompanied by the disappearance of all allergic symptoms.

However, the risk of eliciting side effects (1), which can even be serious although remarkably reduced with the use of either slow-release vaccines or vaccines administered through routes which are alternative to injections, has restricted the use of SIT in the treatment of allergic diseases.

In the last years, attention has been focused on the development of effective, safer vaccines. In particular, an important target is the development of vaccines consisting of mutagenized recombinant proteins, i.e. hypoallergenic variants capable of favourably affecting the natural progression of the disease without causing undesired side effects (2).

*Parietaria* pollen is one of the most important causes of allergy in the Mediterranean area. The two main allergens of this pollen, Par j 1 (whose nucleotide sequence corresponds to GenBank accession number AC X77414) and Par j 2 (AC X95865), are proteins which have a molecular weight of approximately 12 kD, and are partially homologous in their sequences and functions (3, 4, 5).

### DESCRIPTION OF THE INVENTION

It has now been found that the allergenic effect of Par j 1 can be diminished by modifying its sequence through substitution or deletion of one or more amino acid residues.

According to a first aspect, the invention provides a hypoallergenic protein which is a sequence variant of the *Parietaria judaica* major allergen (Par j 1) and which, compared to Par j 1 wild-type (SEQ ID N. 1), has reduced reactivity to IgEs and contains an amino acid sequence which is selected **from SEQ ID N. 6 and SEQ ID N. 7.**

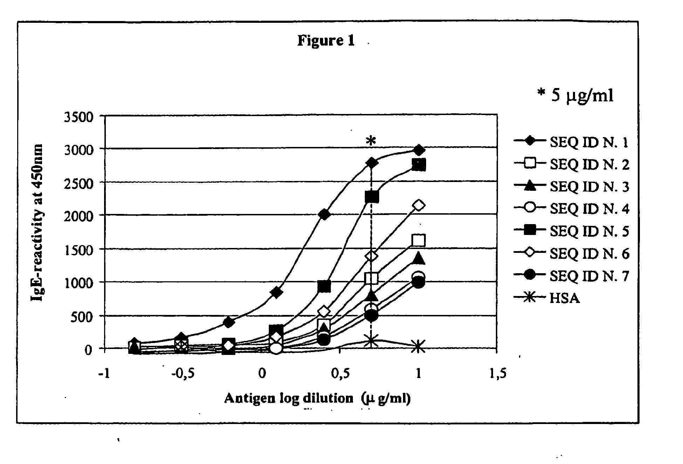

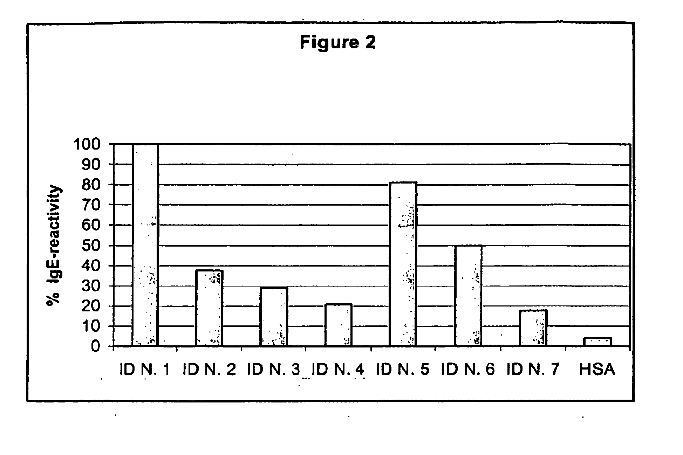

The reactivity to IgEs of the proteins SEQ ID N. 6-7 from a pool of sera of allergic subjects has been tested in an ELISA assay (Fig. 1). At 5 µg/ml, these variants reduced the reactivity to IgEs by an extent of 50% (SEQ ID N. 6) and 82% (SEQ ID N. 7) (Fig. 2), compared to Par j 1 allergen wt (SEQ ID N. 1).

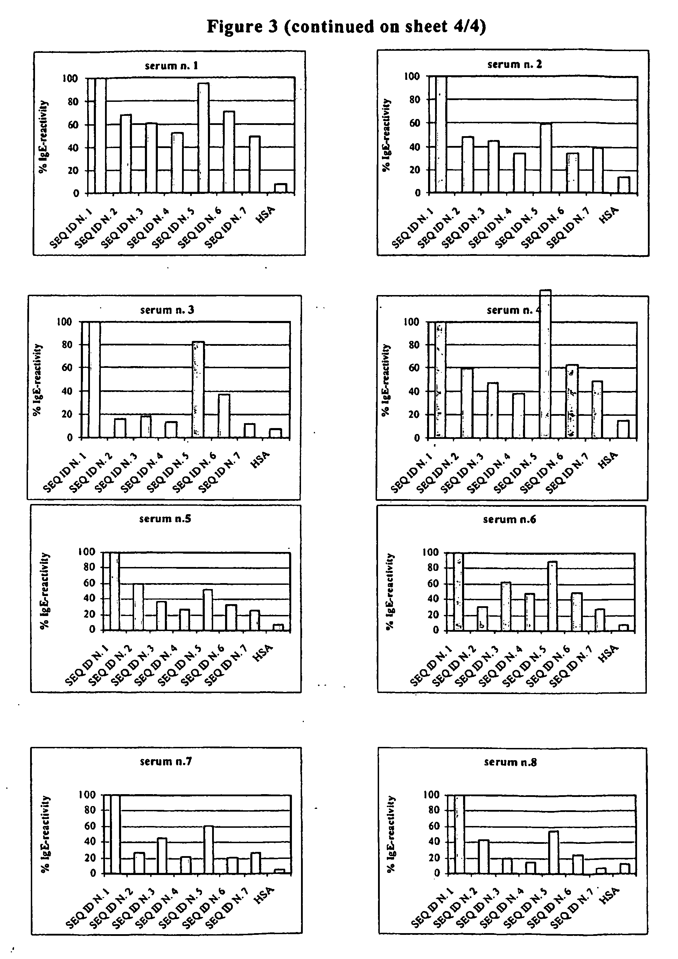

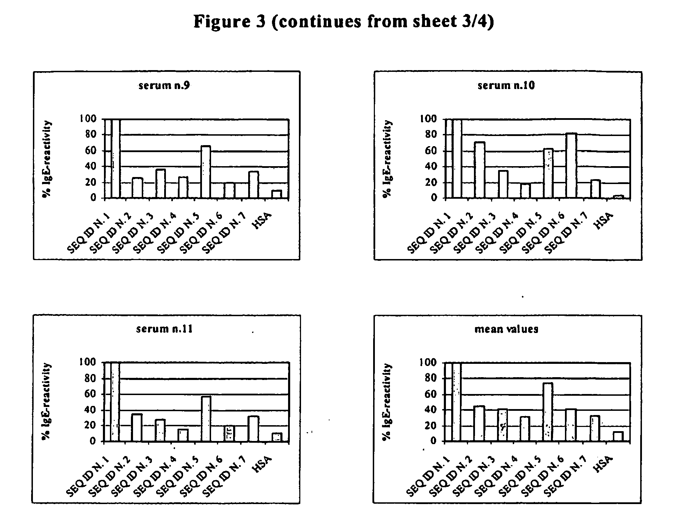

Furthermore, the reactivity to IgEs of SEQ ID N. 6-7 variants in each serum from eleven *Parietaria*-allergic subjects has been tested in an ELISA assay. Also in this case, all the variants showed a reduced allergenicity in the analyzed sera (Tab. 1 and Fig. 3). On average, this was at least a 50% reduction.

In a further aspect, the invention is directed to a nucleic acid molecule coding for a Par j 1 allergen variant herein described, or for a peptide derived therefrom.

Substitution and/or deletion variants according to the invention can be easily prepared by mutagenesis of Par j 1 cDNA sequence (SEQ ID N. 14) using techniques which are known to those skilled in the art.

The cDNA sequences coding for substitution variants of SEQ ID N. 6-7 are reported in SEQ ID N. 12-13.

The invention further refers to an expression vector comprising a nucleic acid molecule which codes for any of the above-defined hypoallergenic variants. Such a vector can be a plasmid, virus, phage or any other vector which is commonly used for genetic engineering purposes, and may comprise, in addition to the nucleic acid molecule of the invention, control elements for the expression in eukaryotic or prokaryotic cells, such as transcription promoters or enhancers, signal sequences or other sequences for transcription regulation.

The invention further comprises a prokaryotic or eukaryotic host cell which is transformed or transfected with the vector of the invention. Prokaryotic cells such as *Escherichia coli* or *Bacillus subtilis,* or eukaryotic cells such as *Saccharomyces cerevisiae* are generally used for vector cloning and cDNA expression.

In addition, hypoallergenic variants according to the invention can be produced as fusion proteins.

Given the reduced reactivity to IgEs, Par j 1 variants according to the present invention can be conveniently used for the preparation of pharmaceutical compositions for the immunotherapy of *Parietaria* pollen-allergic individuals.

In a further aspect the invention is therefore directed to a pharmaceutical composition comprising an effective amount of a Par j 1 hypoallergenic variant, optionally combined with other *Parietaria* allergens, together with pharmaceutically acceptable vehicles or excipients. In a preferred embodiment, the pharmaceutical composition is a vaccine for the prophylactic or therapeutic treatment of allergic diseases, such as bronchial asthma, allergic rhinitis, allergic dermatitis, and allergic conjunctivitis. Principles and methods for vaccination are known to those skilled in the art, and are described, for example, in (6) and (7).

### DESCRIPTION OF THE FIGURES

Figure 1: IgE-reactivity analysis of rPar j 1 allergen and its hypoallergenic variants through an ELISA assay.
Figure 2: IgE-reactivity of a pool of sera to rPar j 1 and its hypoallergenic variants (5 µg/ml).
Figure 3: IgE-reactivity of individual sera to rPar j 1 and its hypoallergenic variants.

The following examples describe the invention in more detail.

### EXAMPLES

Unless otherwise indicated, the methods used in the following examples -are described in Sambrook, Fritsch ET Maniatis "Molecular Cloning. A Laboratory Manual", II ed. vol. 1-2-3, CSH Lab Press, 1989.

### EXAMPLE 1 - Site-specific mutagenesis of the cDNA coding for Par

### i 1 allergen

Site-specific mutagenesis of the cDNA coding for Par j 1 allergen (SEQ ID NO: 14) was carried out by PCR (Polymerase Chain Reaction) amplification of the same cDNA which had been cloned into a prokaryotic vector (pBluescript, GenBank acc. n. X52327). Oligonucleotides used as primers in the PCR reaction (Tab. 1) had the appropriate base substitutions. For each mutagenesis, a complementary oligonucleotide which binds to a corresponding region of the DNA molecule strand (8) was used. Following amplification, the unmodified original template was selectively degraded during an enzymatic digestion catalyzed by the restriction enzyme *Dpn*I. *Escherichia coli* cells were then transformed with the mutagenized molecules. Clones obtained from single bacterial colonies were sequenced by the Sanger method to assess the correct base modification and absence of non-specific mutations in the cDNA.

Tab. 1. Sequences of the oligonucleotides used as primers in site-specific mutagenesis reactions. The bases yielding the mutation are in bold. The underlined bases correspond to a *Bam*HI restriction site, which was used for cloning.

| Oligonucleotide code | Sequence of the oligonucleotide |
|---|---|
| Pj1 23 | cag ggg aaa gag gca gag ccg tca aag |
| Pj1 23.27 | g cag ggg aaa gag gca gag ccg tca gcg ggg tgc tgc |
| Pj1 66 | gac atc gac ggg gca ctc gtc agc gag g |
| Pj1 73 | agc gag gtc ccc gcg cac tgc ggc atc g |
| Pj1 41 | c ggg gag acg gcg acg ggg ccg |
| Pj1 41.42.46 | g gag acg gcg gcg ggg ccg cag gcg gtg cac gct tg |
| Pj1 92 | aac atg gac tgc gcg aca ctt gga gtg g |
| Pj1 121 | gac ccc gcc cac gca gca cgg ttg gag |
| Pj1 138 | ccc gca ccg gaa gca gcc taa gga tcc |

### EXAMPLE 2 - Production of Par j 1 protein and its variants

Par j 1 wt (SEQ ID N. 14) and mutagenized (SEQ ID N. 8-13) cDNAs, linked to a coding sequence coding for six histidines, were expressed in *Escherichia coli* cells according to standard protocols (9, 10) after cloning into an expression vector. Cells were collected by centrifugation and resuspended in a 50 mM NaH₂PO₄, 300 mM NaCl buffer, pH 8. Recombinant proteins were isolated following lysis of bacterial cells by sonication and elimination of cell pellets through centrifugation. Proteins were purified from the supernatant by affinity chromatography using agarose columns bound to nitrilotriacetic acid which chelates nickel ions interacting with the six-histidine portion fused to the allergen.

### EXAMPLE 3 - Characteristics of the sera from allergic subjects

The sera were collected from individuals with a clinical history of seasonal allergy to *Parietaria* pollen and a specific RAST 3+ and 4+ reactivity to *Parietaria* allergens. The sera pool was obtained by gathering together sera with a high level of RAST 4+ class IgEs specific for *Parietaria* allergens.

### EXAMPLE 4 - ELISA analysis of the reactivity of Par j 1 variants to IgEs from a pool of sera

Starting from a 10 µg/ml concentration, 2-fold serial dilutions of normal allergen and its mutagenized variants in 50 mM carbonate/bicarbonate buffer, pH 9.6, were adsorbed on wells of polystyrene plates for ELISA assays by incubation at 4°C for 16 hours. Equivalent amounts of human serum albumin (HSA) were adsorbed as negative controls. Wells were then washed with washing solution (60 mM phosphate buffer, pH 6.5, containing 0.05% Tween-20), and blocked with diluting solution (25% horse serum, 1 mM EDTA, 0.05% Tween 20, 0.01% Thiomersal in 150 mM phosphate buffer, pH 7.4). Equal aliquots (100 µl) of a 1:5 dilution of a pool of human sera with RAST 4+ reactivity were added to each sample and incubated at 25°C for 2 hours. After three washes, the anti-human IgE peroxidase-conjugated antiserum (1:1500 diluted in diluting buffer) was added, followed by incubation at 25°C for 1.5 hours. After three washes, development of the colorimetric reaction was obtained by adding 100 µl of TMB reagent (BioFX Laboratories, Owings Mills, MD) and incubating for 15 minutes at 25°C. The reaction was stopped by adding 100 µl of 1 N HCl, and measured in a spectrophotometer at 450 nm.

### EXAMPLE 5 - ELISA analysis of IgE-reactivity of Par j 1 variants in individual sera

Equivalent amounts (0,2 µg) of normal allergen, its mutagenized variants, and human serum albumin (HSA), the latter being used as a negative control, in 50 mM carbonate/bicarbonate buffer, pH 9.6, were adsorbed on wells of polystyrene plates for ELISA assays by incubation at 4°C for 16 hours. Wells were then washed with washing solution (60 mM phosphate buffer, pH 6.5, containing 0.05% Tween-20), and blocked with diluting solution (25% horse serum, 1 mM EDTA, 0.05% Tween 20, 0.01% Thiomersal in 150 mM phosphate buffer, pH 7.4). Equal aliquots (50 µl) of a 1:5 dilution of human serum with RAST 3+ and 4+ reactivity were added to each sample and incubated at 25°C for 2 hours. After three washes, the anti-human IgE peroxidase-conjugated antiserum (1:1500 diluted in diluting buffer) was added, followed by incubation at 25°C for 1.5 hours. After three washes, the colorimetric reaction was developed by adding 100 µl of TMB reagent (BioFX Laboratories, Owings Mills, MD) and incubating for 15 minutes at 25°C. The reaction was stopped by adding 100 µl of 1 N HCl, and analyzed in a spectrophotometer at 450 nm.

**Tab. 2. IgE-reactivity of individual sera to rPar j 1 and its hypoallergenic variants**

| variant | SEQ ID N. 1 | SEQ ID N. 2 | SEQ ID N. 3 | SEQ ID N. 4 | SEQ ID N. 5 | SEQ ID N. 6 | SEQ ID N. 7 | HSA |
|---|---|---|---|---|---|---|---|---|
| Serum | | | | | | | | |
| **1** | 100,0 | 68,2 | 61,1 | 52,8 | 95,2 | 71,1 | 49,1 | 7,7 |
| **2** | 100,0 | 48,1 | 44,6 | 33,6 | 59,2 | 33,6 | 39,2 | 13,7 |
| **3** | 100,0 | 15,7 | 17,8 | 13,1 | 82,8 | 37,6 | 11,8 | 7,1 |
| **4** | 100,0 | 59,7 | 47,1 | 37,9 | 127,5 | 62,9 | 48,7 | 14,7 |
| **5** | 100,0 | 59,5 | 36,9 | 26,5 | 52,6 | 33,3 | 25,3 | 6.9 |
| **6** | 100,0 | 30,7 | 61,9 | 47,5 | 88,8 | 48,6 | 27,7 | 6,8 |
| **7** | 100,0 | 27,2 | 44,7 | 21,4 | 60,6 | 20,9 | 26,8 | 4,7 |
| **8** | 100,0 | 43,3 | 19,0 | 14,8 | 54,6 | 24,9 | 6,9 | 12,3 |
| **9** | 100,0 | 25,0 | 37,0 | 27,3 | 66,3 | 19,1 | 34,4 | 9,4 |
| **10** | 100,0 | 71,0 | 35,4 | 18,3 | 62,1 | 81,4 | 23,2 | 3,2 |
| **11** | 100,0 | 35,2 | 27,8 | 16,0 | 57,6 | 20,4 | 32,6 | 11,4 |
| Mean IgE reactivity | **100,0** | **45,0** | **41,8** | **31,1** | **74,8** | **41,9** | **32,4** | **12,3** |

### BIBLIOGRAPHY

1. Toubi E., Kessel A., Blant A., Golan T.D., (1999) "Follow-up after systemic adverse reactions of immunotherapy". Allergy, 54(6): 617-620.
2. Akdis C.A., Blaser K., (2000) "Regulation of specific immune response by chemical and structural modifications of allergens". Int. Arch. Allergy Immunol., 121(4): 261-269.
3. Costa M.A., Colombo P., Izzo V., Kennedy D., Venturella S., Cocchiara R., Mistrello G., Falagiani P., Geraci D., (1994). "cDNA cloning, expression and primary structure of Par j I, a major allergen of Parietaria judaica pollen". FEBS Lett., 341:182-186.
4. Duro G., Colombo P., Costa M.A., Izzo V., Porcasi R., Di Fiore R., Locorotondo G., Mirisola M.G., Cocchiara R., Geraci D., (1996). "cDNA cloning, sequence analysis and allergological characterization of Par j 2.0101, a new major allergen of the Parietaria judaica pollen". FEBS Lett., 399: 295-298.
5. Colombo P., Kennedy D., Ramsdale T., Costa M.A., Duro G., Izzo V., Salvadori S., Guerrini R., Cocchiara R., Mirisola M.G., Wood S., Geraci D., (1998). "Identification of an immunodominant IgE epitope of the Parietaria judaica major allergen". J. Immunol., 160: 2780-2785.
6. Paul, (1989), "Fundamental Immunology", Raven press, New York.
7. Cryz, S. J. (1991), "Immunotherapy and Vaccines", VCH Verlagsgesellschaft.
8. Wang W., Malcolm BA. (2002). " Two-stage polymerase chain reaction protocol allowing introduction of multiple mutations, deletions, and insertions, using QuikChange site-directed mutagenesis". Methods Mol Biol.;182: 37-43.
9. Younghee Kim. (2004). "Cloning and Expression of a Lipase Gene from Rice (Oryza sativa cv. Dongjin)". Mol. Cells, 18 (1): 40-45.
10. Asturias JA, Ibarrola I, Eseverri JL, Arilla MC, Gonzales-Rioja R, Martinez A. (2004). "PCR-based cloning and immunological characterization of Parietaria judaica pollen profilin". J Investig Allergol Clin Immunol, 14: 43-48.

## Claims

1. A hypoallergenic protein obtainable by mutagenesis of the *Parietaria judaica* major allergen Par j 1 (SEQ ID NO:1), wherein said protein is selected from the group consisting of SEQ ID NO:6 and SEQ ID NO:7.

2. A nucleic acid molecule coding for a protein according to claim 1.

3. A nucleic acid molecule according to claim 2, which is selected from the group consisting of SEQ ID NO: 12-13.

4. A vector containing the nucleic acid molecule of claims 2 or 3.

5. A host cell containing the vector of claim 4.

6. A pharmaceutical composition comprising an effective amount of a hypoallergenic protein of Parietaria according to claim 1.

7. A composition according to claim 6, in the form of a vaccine.

8. Use of a hypoallergenic protein of Parietaria according to claim 1 for the preparation of a pharmaceutical composition for the prophylactic or therapeutic treatment of allergic diseases caused by pollen of plants of the species Parietaria.

9. Use according to claim 8 for the treatment of bronchial asthma, rhinitis, dermatitis, or allergic conjunctivitis.

## Patentansprüche

1. Ein hypoallergenes Protein erhältlich durch Mutagenese des *Parietaria judaica* Hauptallergens Par j 1 (SEQ ID NO: 1), wobei das Protein aus der Gruppe bestehend aus SEQ ID NO: 6 und SEQ ID NO: 7 ausgewählt ist.

2. Ein Nukleinsäuremolekül kodierend für ein Protein gemäß Anspruch 1.

3. Ein Nukleinsäuremolekül gemäß Anspruch 2, das aus der Gruppe bestehend aus SEQ ID NO: 12-13 ausgewählt ist.

4. Ein Vektor enthaltend das Nukleinsäuremolekül der Ansprüche 2 oder 3.

5. Eine Wirtszelle enthaltend den Vektor des Anspruchs 4.

6. Eine pharmazeutische Zusammensetzung umfassend eine effektive Menge eines hypoallergenen Proteins von *Parietaria* gemäß Anspruch 1.

7. Eine Zusammensetzung gemäß Anspruch 6, in Form eines Impfstoffes.

8. Verwendung eines hypoallergenen Proteins von *Parietaria* gemäß Anspruch 1 für die Herstellung einer pharmazeutischen Zusammensetzung für die prophylaktische oder therapeutische Behandlung von allergischen Erkrankungen, die durch Pollen von Pflanzen der Spezies *Parietaria* verursacht werden.

9. Verwendung gemäß Anspruch 8 für die Behandlung von bronchialem Asthma, Rhinitis, Dermatitis, oder allergischer Konjunktivitis.

## Revendications

1. Une protéine hypoallergénique pouvant être obtenue par mutagenèse de l'allergène principal de *Parietaria judaica* Par j 1 (SEQ ID NO :1), dans laquelle ladite protéine est sélectionnée dans le groupe constitué de SEQ ID NO :6 et SED ID NO :7.

2. Une molécule d'acide nucléique codant pour une protéine selon la revendication 1.

3. Une molécule d'acide nucléique selon la revendication 2, qui est sélectionnée dans le groupe constitué de SEQ ID NO :12-13.

4. Un vecteur contenant une molécule d'acide nucléique des revendications 2 ou 3.

5. Une cellule hôte contenant le vecteur de la revendication 4.

6. Une composition pharmaceutique comprenant une quantité efficace d'une protéine hypoallergénique de Parietaria selon la revendication 1.

7. Une composition selon la revendication 6, sous la forme d'un vaccin.

8. Utilisation d'une protéine hypoallergénique de Parietaria selon la revendication 1 pour la préparation d'une composition pharmaceutique pour le traitement prophylactique ou thérapeutique de maladies allergiques causées par le pollen des plantes des espèces de Parietaria.

9. Utilisation selon la revendication 8 pour le traitement de l'asthme bronchique, des rhinites, des dermatites, ou des conjonctivites allergiques.
